(19) Europäisches Patentamt
European Patent Office
Office européen des brevets

(11) EP 4 317 399 A1

(12) **EUROPEAN PATENT APPLICATION**
published in accordance with Art. 153(4) EPC

(43) Date of publication:
**07.02.2024 Bulletin 2024/06**

(21) Application number: **22781216.1**

(22) Date of filing: **31.03.2022**

(51) International Patent Classification (IPC):
***C12M 1/00*** *(2006.01)* ***G01N 33/48*** *(2006.01)*
***G01N 33/50*** *(2006.01)*

(52) Cooperative Patent Classification (CPC):
**C12M 1/00; G01N 33/48; G01N 33/50**

(86) International application number:
**PCT/JP2022/016441**

(87) International publication number:
**WO 2022/211006 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2021 JP 2021060546**

(71) Applicants:
- **Sanyo Chemical Industries, Ltd.**
**Kyoto-shi, Kyoto 605-0995 (JP)**
- **Egret Lab, Ltd.**
**Tokushima-shi, Tokushima, 770-8079 (JP)**

(72) Inventors:
- **OTA, Koji**
**Kyoto-shi, Kyoto 605-0995 (JP)**
- **ABE, Hideharu**
**Tokushima-shi, Tokushima 770-8501 (JP)**
- **UTE, Koichi**
**Tokushima-shi, Tokushima 770-8501 (JP)**

(74) Representative: **Duncombe, Jessica et al**
**Kilburn & Strode LLP**
**Lacon London**
**84 Theobalds Road**
**London WC1X 8NL (GB)**

(54) **CROSS-LINKED POLYMER, METHOD FOR SEPARATING SUBSTANCE, KIT FOR SEPARATING SUBSTANCE, KIT FOR TESTING FOR DISEASE, AND DEVICE FOR SEPARATING SUBSTANCE**

(57) Provided is a crosslinked polymer useful for separating a substance having a lipid bilayer.

A crosslinked polymer comprising a monomer unit containing an acidic group and/or a neutralized salt group thereof, the crosslinked polymer comprising a compound containing at least one group selected from the group consisting of a cationic group and a hydroxyl group, can be suitably used for separating a substance having a lipid bilayer from a sample of biological origin.

EP 4 317 399 A1

## Description

Technical Field

The present invention relates to a crosslinked polymer and use of the crosslinked polymer.

Background Art

Extracellular vesicles secreted from cells are vesicles that are surrounded by a lipid bilayer and contain proteins, nucleic acids, etc., and function as intercellular communication mediators. Examples of extracellular vesicles include exosomes, microvesicles, apoptotic bodies, and the like. Of these, exosomes (membrane vesicles with a particle size of 30 to 100 nm) are secreted from a variety of cells, and their presence has been confirmed in every body fluid throughout the body, suggesting that exosomes possibly circulate throughout body fluids to transmit information to remote cells. Substances such as proteins or nucleic acids encapsulated in exosomes reflect in real time the condition of the cells that have secreted them, so exosomes are useful as a biomarker for evaluating, for example, the condition of the original organism or its intercellular communication.

Size fractionation using an ultracentrifuge is a widely used method in recovering exosomes from a sample of biological origin. This method separates relatively low-density exosomes from other particles by using, for example, a sucrose density gradient.

Summary of Invention

Technical Problem

However, size fractionation using an ultracentrifuge is unsatisfactory in terms of the purity of recovered exosomes, and the operation takes a long time.

An object of the present invention is to provide a crosslinked polymer useful for separating a substance having a lipid bilayer, such as an exosome.

Solution to Problem

The present inventors conducted extensive research to achieve the object and found that a crosslinked polymer comprising a monomer unit containing an acidic group and/or a neutralized salt group thereof, the crosslinked polymer comprising a compound containing at least one group selected from the group consisting of a cationic group and a hydroxyl group, is useful for separating a substance having a lipid bilayer, such as an exosome. The inventors conducted further research on the basis of this finding and completed the present invention.

The present invention includes the following aspects. Item 1.

A crosslinked polymer for separating a substance having a lipid bilayer from a sample of biological origin, the crosslinked polymer comprising a monomer unit containing an acidic group and/or a neutralized salt group thereof, the crosslinked polymer comprising a compound containing at least one group selected from the group consisting of a cationic group and a hydroxyl group.

Item 2.

The crosslinked polymer according to Item 1, wherein the compound contains an optionally substituted $C_{12-30}$ hydrocarbon group.

Item 3.

The crosslinked polymer according to Item 1 or 2, wherein the compound contains a cationic group.

Item 4.

The crosslinked polymer according to any one of Items 1 to 3, wherein the cationic group is $-N(R^1)_2$ (wherein each $R^1$ independently represents a hydrogen atom or an optionally substituted $C_{1-6}$ hydrocarbon group) or $-N^+(R^2)_3$ (wherein each $R^2$ independently represents a hydrogen atom or an optionally substituted $C_{1-6}$ hydrocarbon group) .

Item 5.

The crosslinked polymer according to any one of Items 1 to 4, wherein the $C_{12-30}$ hydrocarbon group is a $C_{12-20}$ aliphatic hydrocarbon group or a $C_{20-30}$ alicyclic hydrocarbon group.

Item 6.

The crosslinked polymer according to Item 5, wherein the aliphatic hydrocarbon group is an alkyl group or an alkenyl group, and the alicyclic hydrocarbon group is a group having a steroid skeleton.

Item 7.

The crosslinked polymer according to any one of Items 1 to 6, wherein the pH of a physiological saline solution containing the crosslinked polymer in an amount of 0.5 wt% based on the weight of physiological saline is 7 to 8.

Item 8.

The crosslinked polymer according to any one of Items 1 to 7, which has a volume average particle size of 100 to 1000 μm.

Item 9.

The crosslinked polymer according to any one of Items 1 to 8, wherein the substance having a lipid bilayer is an extracellular vesicle.

Item 10.

A method for separating a substance having a lipid bilayer from a sample of biological origin, comprising the following steps (1) and (2):

(1) bringing a sample of biological origin into contact with the crosslinked polymer according to any one of Items 1 to 9 to obtain a polymer gel containing a substance having a lipid bilayer; and
(2) mixing the polymer gel with a salt to recover the substance having a lipid bilayer.

Item 11.

A kit for separating a substance having a lipid bilayer from a sample of biological origin, comprising the crosslinked polymer according to any one of Items 1 to 9 and a salt.

Item 12.

A disease test kit using an extracellular vesicle, comprising the crosslinked polymer according to any one of Items 1 to 9 and a salt.

Item 13.

A device for separating a substance having a lipid bilayer from a sample of biological origin, comprising a means for performing the method according to Item 10.

Item 14.

Use of the crosslinked polymer according to any one of Items 1 to 9 for the separation of a substance having a lipid bilayer from a sample of biological origin.

Advantageous Effects of Invention

The crosslinked polymer of the present invention can be suitably used for separating a substance having a lipid bilayer from a sample of biological origin. The separation method of the present invention increases the purity of

the separated substance having a lipid bilayer and decreases the operation time as compared with, for example, conventional ultracentrifugation. The separation method of the present invention also decreases the number of steps as compared with, for example, conventional ultracentrifugation, and is also excellent in reproducibility. The separated substance having a lipid bilayer is usable in evaluation of the condition of the original organism (e.g., testing or diagnosis). The separated substance having a lipid bilayer is also usable as a carrier for cellularly targeted therapeutic agents.

Description of Embodiments

(A) Crosslinked Polymer

The crosslinked polymer of the present invention comprises a monomer unit containing an acidic group and/or a neutralized salt group thereof.

As used herein, "acidic group" refers to a group containing a dissociable proton. Examples of acidic groups include, but are not limited to, a carboxylic acid group (-COOH), a sulfonic acid group [$-S(=O)_2(OH)$], a sulfuric acid group [$-O-S(=O)_2(OH)$], a phosphonic acid group [$-P(=O)(OH)_2$], a phosphoric acid group [$-O-P(=O)(OH)_2$], and the like.

As used herein, "neutralized salt group of an acidic group" refers to a salt group resulting from neutralization of an acidic group and any cation. Specific examples of cations include a metal (e.g., a monovalent or divalent metal), a cation represented by formula $NR_4^+$ (wherein each R independently represents a hydrogen atom or a optionally substituted hydrocarbon group, and two or three Rs may form a ring together with the adjacent nitrogen atom), and the like.

Examples of monovalent metals include alkali metals, such as lithium, sodium, and potassium; and the like. Examples of divalent metals include alkaline earth metals, such as magnesium, calcium, and barium; lead; zinc; tin; and the like.

In the cation represented by formula $NR_4^+$, when R is an optionally substituted hydrocarbon group, the hydrocarbon group may be, for example, an alkyl group, a cycloalkyl group, an aryl group, or an aralkyl group. Examples of alkyl groups include $C_{1-6}$ alkyl groups, such as a methyl group, an ethyl group, a propyl group (an n-propyl group or an isopropyl group), a butyl group (an n-butyl group, an isobutyl group, a sec-butyl group, or a t-butyl group); and the like. Examples of cycloalkyl groups include $C_{5-14}$ cycloalkyl groups, such as a cyclopentyl group and a cyclohexyl group; and the like. Examples of aryl groups include $C_{6-14}$ aryl groups, such as a phenyl group and a naphthyl group; and the like. Examples of aralkyl groups include $C_{7-14}$ aralkyl groups, such as a benzyl group and a phenethyl group; and the like.

Examples of optional substituents of the hydrocarbon group include a halogen atom, a hydroxyl group, a mercapto group, and the like.

When two or three Rs form a ring together with the adjacent nitrogen atom in the cation represented by formula $NR_4^+$, the ring may be a monocyclic ring, such as a pyridine ring or an imidazole ring, or a fused ring, such as a quinoline ring. The ring may have at least one substituent, and examples of substituents include a halogen atom, a hydroxyl group, an amino group, an alkyl group, a haloalkyl group, a hydroxyalkyl group, an N,N-dialkylamino group, and the like. The number of substituents is, for example, 1, 2, or 3.

Typical examples of monomers containing an acidic group include unsaturated carboxylic acids. The unsaturated carboxylic acids include, for example, unsaturated monocarboxylic acids and unsaturated dicarboxylic acids. Examples of unsaturated monocarboxylic acids include $C_{3-10}$ unsaturated monocarboxylic acids, such as acrylic acid, methacrylic acid, and crotonic acid. Examples of unsaturated dicarboxylic acids (including their anhydrides in the present specification) include $C_{4-10}$ unsaturated dicarboxylic acids, such as maleic acid, fumaric acid, citraconic acid, and itaconic acid, and anhydrides of these.

Examples of other monomers containing an acidic group include (meth)acrylic monomers containing a sulfonic acid group (e.g., (meth)acrylic acid sulfoalkyl esters, such as 2-sulfoethyl (meth)acrylate; and N-sulfoalkyl (meth)acrylic acid amides, such as 2-(meth)acrylamido-2-methylpropane sulfonic acid), (meth)acrylic monomers containing a phosphoric acid group (e.g., (meth)acrylic acid phosphonooxyalkyl esters, such as 2-((meth)acryloyloxy)ethyl phosphate), and the like. As used herein, "(meth)acrylic" means acrylic and/or methacrylic, and "(meth)acryloyloxy" means acryloyloxy and/or methacryloyloxy.

The monomers containing an acidic group may be used singly, or in a combination of two or more.

Typical examples of monomers containing a neutralized salt group of an acidic group include unsaturated carboxylic acid salts. Examples of unsaturated carboxylic acid salts include alkali metal salts (e.g., sodium salts and potassium salts), alkylamine salts (e.g., trialkylamine salts, such as a triethyl amine salt), alkanolamine salts (e.g., dialkanolamine salts, such as a diethanolamine salt, and trialkanolamine salts, such as a triethanolamine salt), ammonium salts, and tetraalkyl ammonium salts (e.g., a tetramethyl ammonium salt and a tetraethyl ammonium salt) of unsaturated carboxylic acids.

Examples of other monomers containing a neutralized salt group of an acidic group include neutralized salts of the above-mentioned (meth)acrylic monomers containing a sulfonic acid group, neutralized salts of the above-mentioned (meth)acrylic monomers containing a phosphoric acid group, and the like. Examples of these neutralized salts

include the alkali metal salts described above.

The monomers containing a neutralized salt group of an acidic group may be used singly, or in a combination of two or more.

The crosslinked polymer of the present invention preferably comprises both a monomer unit containing an acidic group and a monomer unit containing a neutralized salt group of the acidic group. That is, it is preferred that in the crosslinked polymer comprising a monomer unit containing an acidic group, part of the monomer unit containing an acidic group is neutralized and replaced with a monomer unit containing a neutralized salt group of the acidic group. The degree of neutralization (100 × (the number of moles of the neutralized salt group of the acidic group)/(the total number of moles of the acidic group and its neutralized salt group)) is not particularly limited, and may be, for example, 25 mol% or more, 30 mol% or more, 35 mol% or more, 40 mol% or more, 45 mol% or more, or 50 mol% or more, and 90 mol% or less, 85 mol% or less, or 80 mol% or less.

The crosslinked polymer of the present invention may further comprise one or more other monomer units in addition to the monomer unit containing an acidic group and/or a neutralized salt group thereof. Examples of the other monomers include the following monofunctional ethylenically unsaturated monomers:

- esters of unsaturated carboxylic acids, such as acrylic acid and methacrylic acid (e.g., alkyl esters, such as methyl ester, ethyl ester, propyl ester, butyl ester, pentyl ester, hexyl ester, heptyl ester, octyl ester, and 2-ethylhexyl ester; hydroxyalkyl esters, such as 2-hydroxyethyl ester; haloalkyl esters, such as 2,2,2-trifluoroethyl ester; amino alkyl esters, such as 2-amino ethyl ester; (mono- or dialkylamino) alkyl esters, such as 2-(N,N-dimethylamino) ethyl ester; cycloalkyl esters, such as cyclohexyl ester; aryl esters, such as phenyl ester and naphthyl ester; aralkyl esters, such as benzyl ester and phenethyl ester; glycidyl ester; and polyethylene glycol ester)
- unsaturated carboxylic acid amides (e.g., free amides; and N-substituted amides, such as N-monoalkylamide and N,N-dialkylamide)
- unsaturated dicarboxylic acid imides (e.g., maleimide, citraconimide, itaconimide, N-alkyl-substituted form of these, N-cycloalkyl substituted form of these, and N-aryl substituted form of these)
- alkenyl esters of saturated carboxylic acids, such as acetic acid and propionic acid (e.g., vinyl ester and allyl ester)
- esters or amides of unsaturated sulfonic acids (e.g., esters or amides of unsaturated sulfonic acids corresponding to the esters or amides of unsaturated carboxylic acids listed above)
- unsaturated alcohols (e.g., allyl alcohol and propenyl alcohol)
- unsaturated ethers (e.g., alkyl vinyl ethers, such as methyl vinyl ether and ethyl vinyl ether; alkyl allyl ethers, such as methyl allyl ether and ethyl allyl ether; cycloalkyl vinyl ethers, such as cyclohexyl vinyl ether; and glycidyl vinyl ether)
- unsaturated nitriles (e.g., acrylonitrile and methacrylonitrile)
- olefins (e.g., ethylene, propylene, butene, pentene, and hexene)
- aromatic vinyl compounds (e.g., styrene, α-methylstyrene, vinyltoluene, and hydroxystyrene)
- heterocyclic vinyl compounds (e.g., N-vinylpyrrolidone)

The other monomers may be used singly, or in a combination of two or more.

The solubility of the monomer(s) forming the crosslinked polymer in water at 25°C (g/100 g of water) is preferably 1 g or more, and more preferably 5 g or more. Moreover, the monomer(s) forming the crosslinked polymer may be water-miscible.

The crosslinked polymer of the present invention is a polymer having a crosslinked structure (a structure in which constituent atoms of a plurality of linear polymers (polymers of a monomer containing an acidic group and/or a neutralized salt group thereof) are covalently bonded to each other directly or via another atom). In the crosslinked structure, self-crosslinking may be performed when the monomer(s) forming the crosslinked polymer contain a reactive group (e.g., a combination of a monomer containing a carboxyl group and a monomer containing an amino group), or if necessary, crosslinking may be performed using any crosslinking agent.

Examples of methods of crosslinking using a crosslinking agent include the following methods.

1) Method using the following internal crosslinking agent: the monomer(s) described above and an internal crosslinking agent are subjected to a polymerization reaction to directly obtain a crosslinked polymer having a crosslinked structure (e.g., the methods described in JP2003-225565A and JP2005-075982A).
2) Method using the following surface crosslinking agent: a polymer containing the monomer(s) described above as essential monomer(s) is crosslinked with a surface crosslinking agent to obtain a crosslinked polymer (e.g., the methods described in JP3648553B, JP2003-165883A, JP2003-225565A, JP2005-075982A, and JP2005-095759A) .

Examples of crosslinking agents (including internal crosslinking agents and surface crosslinking agents) include, but are not limited to, the following difunctional or higher functional ethylenically unsaturated monomers.

- alkenyl ethers of dihydric or higher alcohols (e.g., alkylene glycols or polyalkylene glycols (the number of carbon atoms of the alkylene group is preferably 2 to 4; the number of repeats of alkylene oxide is preferably 10 to 50), such as ethylene glycol, polyethylene glycol, propylene glycol, butylene glycol, and neopentyl glycol, glycerin, polyglycerin, trimethylol ethane, trimethylol propane, pentaerythritol, dipentaerythritol, and sorbitan) (e.g., vinyl ether and allyl ether)
- esters of unsaturated carboxylic acids (e.g., acrylic acid and methacrylic acid) and dihydric or higher alcohols (e.g., those listed as examples for ethers above)
- alkenyl esters of unsaturated carboxylic acids (e.g., acrylic acid and methacrylic acid) (e.g., vinyl ester and allyl ester)
- alkenyl esters of polycarboxylic acids (e.g., tartaric acid, citric acid, and adipic acid) (e.g., vinyl ester and allyl ester)
- alkenyl esters of isocyanuric acid (e.g., triallyl isocyanate)
- alkylene bis acrylamides (e.g., methylene bis acrylamide)
- alkylene bis methacrylamides (e.g., methylene bis methacrylamide)
- di- or trialkenyl amines (e.g., diallyl amine and triallyl amine)
- aromatic polyvinyl compounds (e.g., divinylbenzene)

The crosslinking agent for use can be a crosslinking agent having at least two functional groups reactive with a substituent (e.g., a carboxy group or a hydroxyl group) of the monomer(s). Examples of such crosslinking agents include polyhydric alcohol glycidyl ethers (e.g., alkylene glycol diglycidyl ethers, such as ethylene glycol diglycidyl ether; and alkane triol diglycidyl ethers or alkane triol triglycidyl ethers, such as glycerin diglycidyl ether), and the like.
The crosslinking agents may be used singly, or in a combination of two or more.
The amount of the crosslinking agent can be suitably selected according to the desired crosslink density, and may be, for example, 0.005 mol% or more or 0.01 mol% or more, and 0.5 mol% or less or 0.4 mol% or less, based on the monomer(s) forming the crosslinked polymer.
The crosslinked polymer of the present invention contains (or supports) a compound containing at least one group selected from the group consisting of a cationic group and a hydroxyl group (hereinafter referred to as "compound X"). That is, the crosslinked polymer of the present invention is in the form of a substance in which the crosslinked polymer and compound X are both present. It is preferred that compound X is present on the surface of the crosslinked polymer of the present invention. It is preferred that compound X is attached or immobilized to the surface of the crosslinked polymer of the present invention by a non-covalent bond, such as a hydrogen bond or an ionic bond. It is preferred that part or all of the surface of the crosslinked polymer of the present invention is coated with compound X. The surface of the crosslinked polymer means the molecular chain of the crosslinked polymer, or when the crosslinked polymer is in the form of, for example, particles or pellets, it means the surface of the particles or pellets of the crosslinked polymer.
The presence of compound X in the crosslinked polymer of the present invention can be identified, for example, by immersing the crosslinked polymer in a suitable solvent (e.g., a hydrocarbon-based solvent, such as hexane), releasing compound X in the solvent, isolating compound X, and subjecting compound X to NMR and gas chromatography.
It is preferred that compound X contains at least one group selected from the group consisting of a cationic group and a hydroxyl group, and an optionally substituted $C_{2-30}$ hydrocarbon group, it is more preferred that compound X contains at least one group selected from the group consisting of a cationic group and a hydroxyl group, and an optionally substituted $C_{12-30}$ hydrocarbon group, and it is more preferred that compound X contains an optionally substituted $C_{12-30}$ hydrocarbon group at one end and at least one group selected from the group consisting of a cationic group and a hydroxyl group at the other end. In compound X, the number of hydrocarbon groups, cationic groups, or hydroxyl groups may be one or two or more. In the present specification, even when compound X contains a cationic group and the cationic group contains a hydroxyl group, it is said that compound X contain a cationic group and a hydroxyl group. Similarly, when compound X contains an optionally substituted $C_{2-30}$ (or $C_{12-30}$) hydrocarbon group and the hydrocarbon group contains a hydroxyl group as a substituent, it is said that compound X contains the hydrocarbon group and a hydroxyl group.
The $C_{12-30}$ hydrocarbon group may be an aliphatic hydrocarbon group, such as an alkyl group or an alkenyl group, an alicyclic hydrocarbon group, such as a cycloalkyl group or a cycloalkenyl group, or an aromatic hydrocarbon group. Examples of optional substituents of the hydrocarbon group include a hydroxyl group, an oxo group (=O), an alkoxy group, an alkenyloxy group, and combinations thereof, and the like. The number of optional substituents of the hydrocarbon group is, for example, 1, 2, 3, 4, or 5.
The $C_{12-30}$ aliphatic hydrocarbon group may be saturated or unsaturated. Examples include $C_{12-20}$ aliphatic hydrocarbon groups, such as a lauryl group, a myristyl group, a palmityl group, a stearyl group, an oleyl group, a linoleyl group, and an arachidyl group; and the like.
For example, lecithin contains $C_{12-30}$ acyl groups, specifically an oleyloyloxy group and a palmitoyloxy group, as optionally substituted $C_{12-30}$ aliphatic hydrocarbon groups. More specifically, lecithin has a diacylglycerol skeleton containing the above acyl groups.

The $C_{12-30}$ alicyclic hydrocarbon group may be, for example, a group having a steroid skeleton. The steroid skeleton is represented by, for example, the following formula (1):

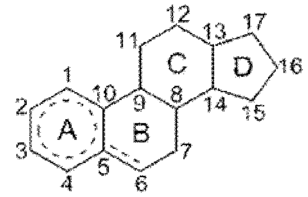

(1)

wherein the dashed line in ring A indicates that any carbon-carbon single bond may be a carbon-carbon double bond, the dashed line in ring B indicates that the carbon-carbon single bond may be a carbon-carbon double bond, and rings A to D may each be substituted. The numbers on the rings are shown for convenience to indicate the positions of substituents.

Examples of optional substituents of rings A to D include a hydroxyl group, an oxo group (=O), an alkyl group (e.g., a $C_{1-4}$ alkyl group, such as methyl), and combinations of these groups (e.g., a hydroxyalkyl group), and the like. The positions of optional substituents are not particularly limited, and examples thereof include the 3-position, 7-position, 10-position, 11-position, 12-position, 13-position, 17-position, and the like.

The skeleton represented by formula (1) includes skeletons represented by the following formulas (1-1) to (1-5).

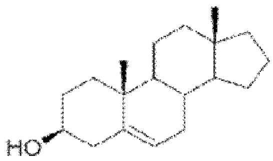

(1-1)

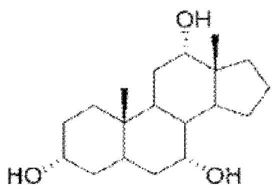

(1-2)

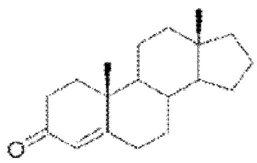

(1-3)

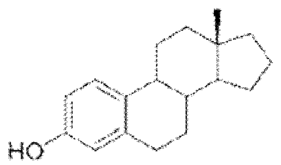

(1-4)

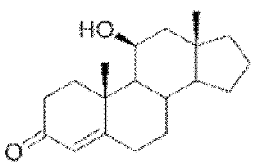

(1-5)

The $C_{12-30}$ alicyclic hydrocarbon group is preferably a $C_{20-30}$ alicyclic hydrocarbon group, more preferably a group in which an optionally substituted alkylene group (e.g., a $C_{2-4}$ alkylene group, such as an ethylene group or a propylene group) is linked to the 17-position of the skeleton represented by formula (1), and even more preferably a group in which an optionally substituted alkylene group (e.g., a $C_{2-4}$ alkylene group, such as an ethylene group or a propylene group) is linked to the 17-position of the skeleton represented by any of formulas (1-1) to (1-5).

Examples of $C_{12-30}$ aromatic hydrocarbon groups include fluorene, anthracene, phenanthrene, tetracene, pyrene, triphenylene, chrysene, tetraphenylene, and the like.

The optionally substituted $C_{12-30}$ hydrocarbon group is preferably a group having a di-$C_{12-30}$ acylglycerol skeleton or a group having a steroid skeleton.

As used herein, "cationic group" includes a group that forms a cation by itself, and a group that does not form a cation by itself but is capable of forming a cation by binding of a proton. Examples of cationic groups include $-N(R^1)_2$ (wherein each $R^1$ independently represents a hydrogen atom or an optionally substituted $C_{1-6}$ hydrocarbon group), $-N^+(R^2)_3$ (wherein each $R^2$ independently represents a hydrogen atom or an optionally substituted $C_{1-6}$ hydrocarbon group), and the like.

In each of $R^1$ and $R^2$ in the above formulas, examples of the $C_{1-6}$ hydrocarbon group include alkyl groups, such as a methyl group, an ethyl group, a propyl group, a butyl group, a pentyl group, and a hexyl group. Examples of optional substituents of the $C_{1-6}$ hydrocarbon group include a hydroxyl group, an oxo group, a carboxylic acid group, a sulfonic acid group, and the like.

The group represented by the above formula $-N(R^1)_2$ is preferably $-NH_2$ or $-N(R^{11})_2$ (wherein $R^{11}$ represents

a $C_{1-5}$ hydroxyalkyl group). Examples of the $C_{1-5}$ hydroxyalkyl group include a hydroxyethyl group, a hydroxypropyl group, a hydroxybutyl group, a hydroxypentyl group (including a pentahydroxypentyl group and the like), and the like.

The group represented by the above formula $-N^+(R^2)_3$ is preferably $-N^+(CH_3)_3$, $-N^+(CH_3)_2C_2H_5$), $-N^+(CH_3)_2(C_3H_7)$, or the like, and these groups may be substituted with, for example, $-CO_2^-$ or $-SO_3^-$.

Preferred examples of compound X include a compound containing a $C_{12-30}$ aliphatic hydrocarbon group and a cationic group (e.g., lecithin and hexadecyltrimethylammonium chloride), a compound containing a $C_{12-30}$ hydrocarbon group and a hydroxyl group (e.g., lauryl alcohol and BIGCHAP), a compound containing a $C_{12-30}$ alicyclic hydrocarbon group, a cationic group, and a hydroxyl group (e.g., CHAPS), a compound containing a cationic group and a hydroxyl group (e.g., triethanolamine), and a polymer containing a cationic group (e.g., polylysine and polyethylenimine), and the like.

The lower limit of the molar concentration of the cationic group per mol of compound X (in particular, when compound X is a polymer containing a cationic group) is preferably 1 mol or more. From the viewpoint of improving the amount of a substance having a lipid bilayer recovered, the lower limit of the molar concentration of the cationic group per mol of compound X is preferably 5 mol or more, and more preferably 10 mol or more. The upper limit of the molar concentration of the cationic group per mol of compound X is preferably 100 mol or less.

The lower limit of the chemical formula weight or number average molecular weight of compound X is preferably 300 or more, and more preferably 500 or more, from the viewpoint of improving the amount of a substance having a lipid bilayer recovered. The upper limit of the chemical formula weight or number average molecular weight of compound X is preferably 10000 or less.

The number average molecular weight of compound X can be measured using gel permeation chromatography (GPC), for example, under the following conditions.

GPC measurement conditions

[1] Device: gel permeation chromatograph (HLC-8120GPC, produced by Tosoh Corporation)
[2] Column: TSKgelG6000PWxl and TSKgelG3000PWxl (both produced by Tosoh Corporation) connected in series
[3] Eluent: solution prepared by dissolving 0.5 wt% sodium acetate in methanol/water=30/70 (volume ratio)
[4] Reference substance: polyethylene glycol (hereinafter abbreviated as "PEG")
[5] Injection conditions: sample concentration: 0.25 wt%; column temperature: 40°C

The crosslinked polymer of the present invention is preferably in the form of particles having a volume average particle size in the range of 50 μm or more, 60 μm or more, 70 μm or more, 80 μm or more, 90 μm or more, 100 μm or more, 110 μm or more, 120 μm or more, 130 μm or more, 140 μm or more, or 150 μm or more, from the viewpoint of reducing impurities (such as albumin) in the separation of a substance having a lipid bilayer.

The crosslinked polymer of the present invention is also preferably in the form of particles having a volume average particle size in the range of 2000 μm or less, 1500 μm or less, 1000 μm or less, 500 um or less, or 300 μm or less, from the viewpoint of improving the amount of a substance having a lipid bilayer recovered. The volume average particle size can be measured by the method described in the Examples shown below.

The pH of a physiological saline solution containing the crosslinked polymer of the present invention in an amount of 0.5 wt% based on the weight of physiological saline (hereinafter abbreviated as "physiological saline solution pH") is not particularly limited. From the viewpoint of reducing negatively charged impurities (such as albumin) in the separation of a substance having a lipid bilayer from a sample of biological origin, the physiological saline solution pH is preferably 6.5 or more, 7 or more, or 7.2 or more, and is preferably 8.5 or less, 8 or less, 7.9 or less, or 7.5 or less. The physiological saline solution pH can be measured by the method described in the Examples shown below.

The physiological saline solution pH can be adjusted according to the degree of neutralization. When the physiological saline solution pH is too low, the physiological saline solution pH tends to be increased by increasing the degree of neutralization. When the physiological saline solution pH is too high, the physiological saline solution pH tends to be decreased by decreasing the degree of neutralization.

The water absorption capacity of the crosslinked polymer of the present invention in physiological saline (physiological saline absorption capacity) is preferably 1 g/g or more, more preferably 3 g/g or more, even more preferably 5 g/g or more, still even more preferably 10 g/g or more, and most preferably 15 g/g or more, from the viewpoint of improving the amount of a substance having a lipid bilayer recovered.

When the water absorption capacity of the crosslinked polymer of the present invention in physiological saline (physiological saline absorption capacity) is too low, the water absorption capacity tends to be increased by increasing the amount of the crosslinking agent.

The upper limit of the water absorption capacity of the crosslinked polymer of the present invention in physiological saline (physiological saline absorption capacity) is not particularly limited, and is, for example, preferably 120 g/g or less, more preferably 100 g/g or less, and particularly preferably 50 g/g or less. The physiological saline absorption capacity can be measured by the method described in the Examples shown below.

When the crosslinked polymer of the present invention contains a cationic group, the molar concentration of the cationic group is preferably $6\times10^{-6}$ to $1\times10^{-4}$ mol/g based on the weight of the crosslinked polymer after drying, from the viewpoint of improving the amount of a substance having a lipid bilayer recovered.

The weight of the crosslinked polymer after drying can be measured, for example, by the following method.

1 g of the crosslinked polymer is placed on a Petri dish, covered with filter paper, and heat-dried in a circulating dryer at 130°C for 60 minutes, and the weight of the residue after heat drying is defined as the weight of the crosslinked polymer after drying.

Since compound X contained in the crosslinked polymer of the present invention has excellent affinity for substances having a lipid bilayer, the crosslinked polymer of the present invention can be suitably used for the separation of substances having a lipid bilayer from samples of biological origin. Examples of substances having a lipid bilayer include extracellular vesicles, such as exosomes.

The crosslinked polymer of the present invention can be produced, for example, by a method including the step of polymerizing a composition containing one or more monomers, a crosslinking agent, a solvent, an initiator, and if necessary, a neutralizer (e.g., solution polymerization, emulsion polymerization, or suspension polymerization), the step of drying the resulting polymer, and if necessary, the step of classifying the polymer.

## (B) Method for Separating Substance Having Lipid Bilayer from Sample of Biological Origin

The method for separating a substance having a lipid bilayer from a sample of biological origin, comprising the following steps (1) and (2):

(1) bringing a sample of biological origin into contact with a crosslinked polymer to obtain a polymer gel containing a substance having a lipid bilayer; and
(2) mixing the polymer gel with a salt to recover the substance having a lipid bilayer.

The sample of biological origin used in step (1) is not particularly limited as long as it contains a substance having a lipid bilayer. Examples of samples of biological origin include body fluids, such as human or animal blood, plasma, serum, lacrimal fluid, saliva, breast milk, pleural effusion, peritoneal fluid, amniotic fluid, cerebrospinal fluid, and urine; liquidized organs, tissues (e.g., hair, nails, skin, muscles, or nerves), or cells (including a cell culture solution and a supernatant thereof); extracts from plants; and the like. The samples of biological origin may be used singly or in a combination of two or more. The sample of biological origin is preferably a body fluid, and more preferably blood, plasma, serum, or urine.

The crosslinked polymer used in step (1) may be the same as the crosslinked polymer described in section "(A) Crosslinked Polymer" above. The amount of the crosslinked polymer used is, for example, preferably 1 part by mass or more, 5 parts by mass or more, or 10 parts by mass or more, and is preferably 2000 parts by mass or less, 1500 parts by mass or less, or 1000 parts by mass or less, per 100 parts by mass of the sample of biological origin.

The temperature at which a sample of biological origin is brought into contact with the crosslinked polymer is, for example, 1 to 35°C, and preferably 5 to 30°C. After contact, it is preferable to allow the sample to stand for a predetermined time (e.g., 1 hour or more or 2 hours or more, or 5 hours or less or 4 hours or less).

Due to step (1), a substance having a lipid bilayer can be separated in the polymer gel.

The salt used in step (2) is not particularly limited as long as the salt can discharge the substance having a lipid bilayer contained in the polymer gel. Examples of salts include metal salts (e.g., a monovalent or divalent metal salt).

Examples of monovalent metal salts include alkali metal salts, such as sodium salts and potassium salts. Examples of divalent metal salts include alkaline earth metal salts, such as magnesium salts, calcium salts, and barium salts.

The counter anion of the salt can be, for example, but is not limited to, a halide ion, such as a chloride ion or a bromide ion.

The salts may be used singly, or in a combination of two or more. The salt is preferably at least one member selected from the group consisting of sodium chloride and magnesium chloride.

The amount of the salt used can be, for example, 1 part by mass or more, 5 parts by mass or more, or 10 parts by mass or more, and 2000 parts by mass or less, 1500 parts by mass or less, or 1000 parts by mass or less, per 100 parts by mass of the polymer gel.

The temperature at which the polymer gel is mixed with the salt is preferably, for example, 1 to 35°C, or 5 to 30°C. After being mixed, the mixture is preferably allowed to stand for a predetermined time (e.g., 1 hour or more or 2 hours or more, or 5 hours or less or 4 hours or less). The mixture of the polymer gel and the salt may be dialyzed as

necessary.

Due to step (2), the substance having a lipid bilayer contained in the polymer gel can be recovered at a high recovery rate. Examples of substances having a lipid bilayer to be recovered include extracellular vesicles, such as exosomes.

The method of the present invention increases the purity of the recovered substance having a lipid bilayer (the amount of foreign matter being decreased), and decreases the operation time as compared with conventional ultracentrifugation.

(C) Kit for Separating Substance Having Lipid Bilayer from Sample of Biological Origin

The present invention includes a kit for separating a substance having a lipid bilayer from a sample of biological origin, comprising a crosslinked polymer and a salt. The configuration of the kit preferably corresponds to that in section "(B) Method for Separating Substance Having Lipid Bilayer from Sample of Biological Origin" above. The kit of the present invention may further contain an instruction manual on the method for separating a substance having a lipid bilayer from a sample of biological origin, an instrument for the separation, and the like.

(D) Disease Test Kit Using Extracellular Vesicle

The present invention includes a disease test (or disease diagnosis) kit using an extracellular vesicle, comprising a crosslinked polymer and a salt.

The test subject may be a healthy subject or a patient. Examples of diseases include lifestyle-related diseases, chronic kidney disease, neurological diseases, immune disorders, cancers, infections, degenerative diseases, and the like.

The kit may be, for example, for separating an extracellular vesicle from a sample (e.g., a body fluid, such as blood, plasma, serum, lacrimal fluid, saliva, breast milk, pleural effusion, peritoneal fluid, amniotic fluid, cerebrospinal fluid, or urine) derived from a test subject and conducting a disease test based on the inclusions of the extracellular vesicle. The kit may be a kit modified to contain a crosslinked polymer and a salt as reagents for separating an extracellular vesicle from a sample derived from a test subject in a known disease test kit using an extracellular vesicle.

The crosslinked polymer and salt in the kit may be the same as the crosslinked polymer and salt described in section "(B) Method for Separating Substance Having Lipid Bilayer from Sample of Biological Origin" above, respectively. The kit may further contain an instruction manual on the method for separating an extracellular vesicle from a sample derived from a test subject, an instrument for the separation, and the like.

(E) Device for Separating Substance Having Lipid Bilayer from Sample of Biological Origin

The present invention includes a device for separating a substance having a lipid bilayer from a sample of biological origin, comprising a means for performing "(B) Method for Separating Substance Having Lipid Bilayer from Sample of Biological Origin," described above.

## Examples

The following describes the present invention in detail with reference to Examples. However, the present invention is not limited to these Examples.

### Method for Measuring Physiological Saline Solution pH

In a cylindrical 100-mL beaker with a diameter of 50 mm, physiological saline (sodium chloride concentration: 0.9 wt%) was added to 0.5 g of a measurement sample to make a total amount of 100 g. The mixture was stirred at 60 rpm with a stir bar (length: 30 mm) at 25°C for 30 minutes and then allowed to stand at 25°C for 1 minute. Thereafter, the pH of the supernatant at 25°C was measured with a pH meter and defined as the physiological saline solution pH.

### Method for Measuring Physiological Saline Absorption Capacity

1 g of a measurement sample was placed in a tea bag (length: 20 cm; width: 10 cm) made of nylon mesh with an opening of 63 μm (JIS Z8801-1:2006), and immersed in 1000 ml of physiological saline (sodium chloride concentration: 0.9 wt%) without stirring for 1 hour. The tea bag was then hung for 15 minutes for drainage. Thereafter, the weight of the tea bag (h1) was measured, and the physiological saline absorption capacity was determined by using the following formula.

Physiological saline absorption capacity (g/g) = (h1) - (h2)

The temperatures of the physiological saline used and the measurement atmosphere were 25°C ± 2°C. The weight of the tea bag was measured in the same manner as described above, except that the measurement sample was not used, and was defined as (h2) .

Production Example 1: Production of Crosslinked Polymer (A-1)

In a 1-L beaker, 116.5 g of acrylic acid, 272.2 g of ion-exchanged water, and 1.5 g of ethylene glycol diglycidyl ether (1.3 wt%/acrylic acid) as a crosslinking agent were placed and mixed to dissolve the crosslinking agent. While the beaker was cooled in an ice bath, 96.1 g of a 48.5 wt% sodium hydroxide aqueous solution was added to neutralize a portion (72 mol%) of the acrylic acid. After the neutralized monomer solution was cooled to 5°C, 9.3 g of a 2 wt% potassium persulfate aqueous solution was added as a polymerization initiator to obtain a monomer aqueous solution.
In a 2-L separable flask equipped with a stirrer and a condenser, 1434 g of cyclohexane and 7.1 g of Rheodol SP-S10V (produced by Kao Corporation, sorbitan monostearate) as a dispersing agent were placed, heated to an internal temperature of 60°C using a hot-water bath, and stirred to dissolve the dispersing agent in the cyclohexane.
After nitrogen was fed to the solution in the separable flask to reduce the amount of dissolved oxygen in the cyclohexane to 0.1 ppm or less, 350 g of the monomer aqueous solution was added dropwise using a dropping funnel while stirring with the stirrer, and reversed-phase suspension polymerization was performed at a polymerization temperature of 80°C. Further, after the completion of dropwise addition of the monomer aqueous solution, heating was further performed for 2 hours to complete the suspension polymerization, thereby obtaining a spherical hydrogel in the cyclohexane. After the rotation of the stirrer was stopped, and the produced hydrogel was allowed to sediment, the cyclohexane was removed by decantation, and the remaining hydrogel was washed with cyclohexane several times to remove the dispersing agent attached to the hydrogel. The resulting spherical hydrogel was spread on release paper and dried in a vacuum dryer (degree of reduced pressure: 10000 to 20000 Pa) at 130°C for 1 hour to obtain a crosslinked polymer (X-1).
The crosslinked polymer (X-1) was adjusted to have a particle size of 150 to 300 μm using sieves with openings of 150 and 300 um to obtain a crosslinked polymer (A-1).
The volume average particle size of the crosslinked polymer (A-1) was measured with a particle analyzer (CAMSIZER XT produced by Retsch) and found to be 220 um. The physiological saline absorption capacity was 26 g/g.

Production Example 2: Production of Crosslinked Polymer (A-2)

A crosslinked polymer (X-2) was obtained in the same manner as in the production method for the crosslinked polymer (X-1) in Production Example 1, except that the amount of ethylene glycol diglycidyl ether was 3.0 g (2.6 wt%/acrylic acid).
The crosslinked polymer (X-2) was adjusted to have a particle size of 150 to 300 um using sieves with openings of 150 and 300 um to obtain a crosslinked polymer (A-2).
The volume average particle size of the crosslinked polymer (A-2) was measured with a particle analyzer (CAMSIZER XT produced by Retsch) and found to be 200 um. The physiological saline absorption capacity was 16 g/g.

Production Example 3: Production of Crosslinked Polymer (A-3)

A crosslinked polymer (X-3) was obtained in the same manner as in the production method for the crosslinked polymer (X-1) in Production Example 1, except that the amount of ethylene glycol diglycidyl ether was 0.58 g (0.5 wt%/acrylic acid).
The crosslinked polymer (X-3) was adjusted to have a particle size of 300 to 500 um using sieves with openings of 300 and 500 um to obtain a crosslinked polymer (A-3).
The volume average particle size of the crosslinked polymer (A-3) was measured with a particle analyzer (CAMSIZER XT produced by Retsch) and found to be 410 um. The physiological saline absorption capacity was 35 g/g.

Production Example 4: Production of Crosslinked Polymer (A-4)

In a 2-L beaker, 300 g of acrylic acid, 700 g of ion-exchanged water, and 2.0 g of trimethylolpropane triacrylate (0.66 wt%/acrylic acid) as a crosslinking agent were placed and mixed with stirring to prepare an acrylic acid aqueous solution. The acrylic acid aqueous solution was cooled to 3°C.
The acrylic acid aqueous solution was poured in a 2-L adiabatic polymerization tank, and nitrogen was fed to

the acrylic acid aqueous solution to reduce the amount of dissolved oxygen in the acrylic acid aqueous solution to 0.1 ppm or less. To the adiabatic polymerization tank, 1.2 g of a 1 wt% hydrogen peroxide aqueous solution, 2.3 g of a 2 wt% L-ascorbic acid aqueous solution, and 4.5 g of a 2 wt% 2,2'-azobis[2-methyl-N-(2-hydroxyethyl)-propionamide] aqueous solution (produced by Wako Pure Chemical Industries, Ltd., trade name: VA-086) were added, and feeding of nitrogen to the acrylic acid aqueous solution was continued until polymerization started. After confirming that the viscosity of the acrylic acid aqueous solution started to increase after the start of polymerization, feeding of nitrogen was stopped, and the polymerization was performed for 6 hours. The temperature of the acrylic acid aqueous solution was measured with a point-type thermometer, and the maximum temperature achieved was 94°C.

The block-shaped crosslinked hydrogel was taken out from the adiabatic polymerization tank and cut into noodles with a thickness of 3 to 10 mm by using a small meat chopper (produced by Royal), and then 292 g of a 40 wt% sodium hydroxide (guaranteed reagent) aqueous solution (corresponding to a degree of neutralization of acrylic acid of 70 mol%) was added to neutralize the hydrogel. Subsequently, 45 g of a 10 wt% sodium sulfite aqueous solution was added, and the hydrogel was uniformly kneaded with the small meat chopper.

The neutralized cut gel was dried with a through-flow band dryer (150°C; wind speed: 2 m/sec) to obtain a dried product. The dried product was pulverized with a juicer-mixer (OSTERIZER BLENDER produced by Oster) to obtain a crosslinked polymer (X-4).

The crosslinked polymer (X-4) was adjusted to have a particle size of 150 to 300 μm using sieves with openings of 150 and 300 μm to obtain a crosslinked polymer (A-4).

The volume average particle size of the crosslinked polymer (A-4) was measured with a particle analyzer (CAMSIZER XT produced by Retsch) and found to be 222 um. The physiological saline absorption capacity was 36 g/g.

Production Example 5: Production of Crosslinked Polymer (A-5)

A crosslinked polymer (A-5) was obtained by adjusting the crosslinked polymer (X-4) obtained in Production Example 4 to have a particle size of 90 to 150 μm using sieves with openings of 90 and 150 um.

The volume average particle size of the crosslinked polymer (A-5) was measured with a particle analyzer (CAMSIZER XT produced by Retsch) and found to be 125 um. The physiological saline absorption capacity was 33 g/g.

Production Example 6: Production of Crosslinked Polymer (A-6)

A crosslinked polymer (A-6) was obtained by adjusting the crosslinked polymer (X-4) obtained in Production Example 4 to have a particle size of 500 to 710 μm using sieves with openings of 500 and 710 um.

The volume average particle size of the crosslinked polymer (A-6) was measured with a particle analyzer (CAMSIZER XT produced by Retsch) and found to be 630 um. The physiological saline absorption capacity was 38 g/g.

Production Example 7: Production of Crosslinked Polymer (A-7)

A crosslinked polymer (X-7) was obtained in the same manner as in the production method for the crosslinked polymer (X-1) in Production Example 1, except that the amount of ethylene glycol diglycidyl ether was 6 g (5.2 wt%/acrylic acid).

The crosslinked polymer (X-7) was adjusted to have a particle size of 150 to 300 μm using sieves with openings of 150 and 300 μm to obtain a crosslinked polymer (A-7).

The volume average particle size of the crosslinked polymer (A-7) was measured with a particle analyzer (CAMSIZER XT produced by Retsch) and found to be 220 um. The physiological saline absorption capacity was 3 g/g.

Production Example 8: Production of Crosslinked Polymer (A-8)

A crosslinked polymer (X-8) was obtained in the same manner as in the production method for the crosslinked polymer (X-1) in Production Example 1, except that the amount of ethylene glycol diglycidyl ether was 0.2 g (0.17 wt%/acrylic acid).

The crosslinked polymer (X-8) was adjusted to have a particle size of 150 to 300 μm using sieves with openings of 150 and 300 μm to obtain a crosslinked polymer (A-8).

The volume average particle size of the crosslinked polymer (A-8) was measured with a particle analyzer (CAMSIZER XT produced by Retsch) and found to be 220 um. The physiological saline absorption capacity was 100 g/g.

Production Example 9: Production of Crosslinked Polymer (A-9)

A crosslinked polymer (A-9) was obtained by adjusting the crosslinked polymer (X-1) obtained in Production

Example 1 to have a particle size of 100 to 200 μm using sieves with openings of 100 and 200 um.

The volume average particle size of the crosslinked polymer (A-9) was measured with a particle analyzer (CAMSIZER XT produced by Retsch) and found to be 150 um. The physiological saline absorption capacity was 21 g/g.

Production Example 10: Production of Crosslinked Polymer (A-10)

A crosslinked polymer (A-10) was obtained by adjusting the crosslinked polymer (X-1) obtained in Production Example 1 to have a particle size of 250 to 350 μm using sieves with openings of 250 and 350 μm.

The volume average particle size of the crosslinked polymer (A-10) was measured with a particle analyzer (CAMSIZER XT produced by Retsch) and found to be 300 um. The physiological saline absorption capacity was 24 g/g.

Production Example 11: Production of Crosslinked Polymer (A-11)

A crosslinked polymer (A-11) was obtained by adjusting the crosslinked polymer (X-4) obtained in Production Example 4 to have a particle size of 250 to 350 μm using sieves with openings of 250 and 350 μm.

The volume average particle size of the crosslinked polymer (A-11) was measured with a particle analyzer (CAMSIZER XT produced by Retsch) and found to be 288 um. The physiological saline absorption capacity was 34 g/g.

Production Example 12: Production of Crosslinked Polymer (B-1-1)

To 50 g of the crosslinked polymer (A-1), 28 g of a 50 wt% potassium carbonate aqueous solution was added by spraying through a spray nozzle, and the mixture was uniformly mixed, heated at 130°C for 30 minutes, and cooled to room temperature to obtain a crosslinked polymer (B-1-1). The physiological saline solution pH of the crosslinked polymer (B-1-1) was measured and found to be 7.5.

Production Example 13: Production of Crosslinked Polymer (B-1-2)

A crosslinked polymer (B-1-2) was obtained in the same manner as in Production Example 12, except that the amount of the 50 wt% potassium carbonate aqueous solution was 20 g. The physiological saline solution pH of the crosslinked polymer (B-1-2) was measured and found to be 7.2.

Production Example 14: Production of Crosslinked Polymer (B-1-3)

A crosslinked polymer (B-1-3) was obtained in the same manner as in Production Example 12, except that the amount of the 50 wt% potassium carbonate aqueous solution was 32 g. The physiological saline solution pH of the crosslinked polymer (B-1-3) was measured and found to be 7.9.

Production Example 15: Production of Crosslinked Polymer (B-1-4)

A crosslinked polymer (B-1-4) was obtained in the same manner as in Production Example 12, except that the amount of the 50 wt% potassium carbonate aqueous solution was 25 g. The physiological saline solution pH of the crosslinked polymer (B-1-4) was measured and found to be 7.3.

Production Example 16: Production of Crosslinked Polymer (B-2-1)

A crosslinked polymer (B-2-1) was obtained in the same manner as in Production Example 12, except that the crosslinked polymer (A-2) was used in place of the crosslinked polymer (A-1). The physiological saline solution pH of the crosslinked polymer (B-2-1) was measured and found to be 7.4.

Production Example 17: Production of Crosslinked Polymer (B-3-1)

A crosslinked polymer (B-3-1) was obtained in the same manner as in Production Example 13, except that the crosslinked polymer (A-3) was used in place of the crosslinked polymer (A-1). The physiological saline solution pH of the crosslinked polymer (B-3-1) was measured and found to be 7.1.

Production Example 18: Production of Crosslinked Polymer (B-3-2)

A crosslinked polymer (B-4-1) was obtained in the same manner as in Production Example 12, except that the

crosslinked polymer (A-4) was used in place of the crosslinked polymer (A-1), and 12.5 g of 48 wt% sodium hydroxide was used in place of 28 g of the 50 wt% potassium carbonate aqueous solution. The physiological saline solution pH of the crosslinked polymer (B-4-1) was measured and found to be 7.5.

Production Example 19: Production of Crosslinked Polymer (B-5-1)

A crosslinked polymer (B-5-1) was obtained in the same manner as in Production Example 12, except that the crosslinked polymer (A-5) was used in place of the crosslinked polymer (A-1), and 12.5 g of 48 wt% sodium hydroxide was used in place of 28 g of the 50 wt% potassium carbonate aqueous solution. The physiological saline solution pH of the crosslinked polymer (B-5-1) was measured and found to be 7.4.

Production Example 20: Production of Crosslinked Polymer (B-6-1)

A crosslinked polymer (B-6-1) was obtained in the same manner as in Production Example 12, except that the crosslinked polymer (A-6) was used in place of the crosslinked polymer (A-1), and 15.6 g of 48 wt% sodium hydroxide was used in place of 28 g of the 50 wt% potassium carbonate aqueous solution. The physiological saline solution pH of the crosslinked polymer (B-6-1) was measured and found to be 7.9.

Production Example 21: Production of Crosslinked Polymer (B-7-1)

A crosslinked polymer (B-7-1) was obtained in the same manner as in Production Example 12, except that the crosslinked polymer (A-7) was used in place of the crosslinked polymer (A-1). The physiological saline solution pH of the crosslinked polymer (B-7-1) was measured and found to be 7.4.

Production Example 22: Production of Crosslinked Polymer (B-8-1)

A crosslinked polymer (B-8-1) was obtained in the same manner as in Production Example 12, except that the crosslinked polymer (A-8) was used in place of the crosslinked polymer (A-1). The physiological saline solution pH of the crosslinked polymer (B-8-1) was measured and found to be 7.4.

Production Example 23: Production of Crosslinked Polymer (B-9-1)

A crosslinked polymer (B-9-1) was obtained in the same manner as in Production Example 12, except that the crosslinked polymer (A-9) was used in place of the crosslinked polymer (A-1). The physiological saline solution pH of the crosslinked polymer (B-9-1) was measured and found to be 7.4.

Production Example 24: Production of Crosslinked Polymer (B-10-1)

A crosslinked polymer (B-10-1) was obtained in the same manner as in Production Example 12, except that the crosslinked polymer (A-10) was used in place of the crosslinked polymer (A-1). The physiological saline solution pH of the crosslinked polymer (B-10-1) was measured and found to be 7.4.

Production Example 25: Production of Crosslinked Polymer (B-11-1)

A crosslinked polymer (B-11-1) was obtained in the same manner as in Production Example 12, except that the crosslinked polymer (A-11) was used in place of the crosslinked polymer (A-1). The physiological saline solution pH of the crosslinked polymer (B-11-1) was measured and found to be 7.4.

Example 1: Production of Crosslinked Polymer (C-1-1-a)

To 10 g of the crosslinked polymer (B-1-1), 2 g of a 5 wt% lecithin (produced by Fujifilm Wako Pure Chemical Corporation; derived from soybean) cyclohexane solution was added dropwise with a dropper, and the mixture was mixed uniformly, heated at 130°C for 30 minutes, and cooled to room temperature to obtain a crosslinked polymer (C-1-1-a).

The volume average particle size of the crosslinked polymer (C-1-1-a) was measured with a particle analyzer (CAMSIZER XT produced by Retsch) and found to be 220 um. The physiological saline absorption capacity was 22 g/g. The physiological saline solution pH was measured and found to be 7.4.

Example 2: Production of Crosslinked Polymer (C-1-2-a)

A crosslinked polymer (C-1-2-a) was obtained in the same manner as in Example 1, except that the crosslinked polymer (B-1-2) was used in place of the crosslinked polymer (B-1-1).

The volume average particle size of the crosslinked polymer (C-1-2-a) was measured with a particle analyzer (CAMSIZER XT produced by Retsch) and found to be 218 um. The physiological saline absorption capacity was 23 g/g. The physiological saline solution pH was measured and found to be 7.1.

Example 3: Production of Crosslinked Polymer (C-1-3-a)

A crosslinked polymer (C-1-3-a) was obtained in the same manner as in Example 1, except that the crosslinked polymer (B-1-3) was used in place of the crosslinked polymer (B-1-1).

The volume average particle size of the crosslinked polymer (C-1-3-a) was measured with a particle analyzer (CAMSIZER XT produced by Retsch) and found to be 218 um. The physiological saline absorption capacity was 19 g/g. The physiological saline solution pH was measured and found to be 7.9.

Example 4: Production of Crosslinked Polymer (C-1-4-a)

A crosslinked polymer (C-1-4-a) was obtained in the same manner as in Example 1, except that the crosslinked polymer (B-1-4) was used in place of the crosslinked polymer (B-1-1).

The volume average particle size of the crosslinked polymer (C-1-4-a) was measured with a particle analyzer (CAMSIZER XT produced by Retsch) and found to be 218 um. The physiological saline absorption capacity was 22 g/g. The physiological saline solution pH was measured and found to be 7.2.

Example 5: Production of Crosslinked Polymer (C-2-1-a)

A crosslinked polymer (C-2-1-a) was obtained in the same manner as in Example 1, except that the crosslinked polymer (B-2-1) was used in place of the crosslinked polymer (B-1-1), and the amount of the 5 wt% lecithin (produced by Fujifilm Wako Pure Chemical Corporation; derived from soybean) cyclohexane solution added dropwise was 3 g.

The volume average particle size of the crosslinked polymer (C-2-1-a) was measured with a particle analyzer (CAMSIZER XT produced by Retsch) and found to be 201 um. The physiological saline absorption capacity was 11 g/g. The physiological saline solution pH was measured and found to be 7.4.

Example 6: Production of Crosslinked Polymer (C-2-1-b)

A crosslinked polymer (C-2-1-b) was obtained in the same manner as in Example 1, except that the crosslinked polymer (B-2-1) was used in place of the crosslinked polymer (B-1-1), and 1 g of a 5 wt% BIGCHAP (produced by Dojindo Laboratories; N,N-bis(3-D-gluconamidopropyl)cholamide) cyclohexane solution was used in place of 2 g of the 5 wt% lecithin (produced by Fujifilm Wako Pure Chemical Corporation; derived from soybean) cyclohexane solution.

The volume average particle size of the crosslinked polymer (C-2-1-b) was measured with a particle analyzer (CAMSIZER XT produced by Retsch) and found to be 207 um. The physiological saline absorption capacity was 11 g/g. The physiological saline solution pH was measured and found to be 7.4.

Example 7: Production of Crosslinked Polymer (C-3-1-c)

A crosslinked polymer (C-3-1-c) was obtained in the same manner as in Example 1, except that the crosslinked polymer (B-3-1) was used in place of the crosslinked polymer (B-1-1), and 1 g of a 5 wt% hexadecyltrimethylammonium chloride (produced by Fujifilm Wako Pure Chemical Corporation; HDTMA-Cl) cyclohexane solution was used in place of 2 g of the 5 wt% lecithin (produced by Fujifilm Wako Pure Chemical Corporation; derived from soybean) cyclohexane solution.

The volume average particle size of the crosslinked polymer (C-3-1-c) was measured with a particle analyzer (CAMSIZER XT produced by Retsch) and found to be 420 um. The physiological saline absorption capacity was 32 g/g. The physiological saline solution pH was measured and found to be 7.0.

Example 8: Production of Crosslinked Polymer (C-4-1-d)

A crosslinked polymer (C-4-1-d) was obtained in the same manner as in Example 1, except that the crosslinked polymer (B-4-1) was used in place of the crosslinked polymer (B-1-1), and 2 g of a 5 wt% CHAPS (produced by Fujifilm

Wako Pure Chemical Corporation; 3-[(3-cholamidopropyl)dimethylammonio]propanesulfonate) cyclohexane solution was used in place of 2 g of the 5 wt% lecithin (produced by Fujifilm Wako Pure Chemical Corporation; derived from soybean) cyclohexane solution.

The volume average particle size of the crosslinked polymer (C-4-1-d) was measured with a particle analyzer (CAMSIZER XT produced by Retsch) and found to be 222 um. The physiological saline absorption capacity was 32 g/g. The physiological saline solution pH was measured and found to be 7.0.

Example 9: Production of Crosslinked Polymer (C-4-1-e)

A crosslinked polymer (C-4-1-e) was obtained in the same manner as in Example 1, except that the crosslinked polymer (B-4-1) was used in place of the crosslinked polymer (B-1-1), and 2 g of a 5 wt% lauryl alcohol (produced by Tokyo Chemical Industry Co., Ltd.) cyclohexane solution was used in place of 2 g of the 5 wt% lecithin (produced by Fujifilm Wako Pure Chemical Corporation; derived from soybean) cyclohexane solution.

The volume average particle size of the crosslinked polymer (C-4-1-e) was measured with a particle analyzer (CAMSIZER XT produced by Retsch) and found to be 228 um. The physiological saline absorption capacity was 28 g/g. The physiological saline solution pH was measured and found to be 7.4.

Example 10: Production of Crosslinked Polymer (C-5-1-d)

A crosslinked polymer (C-5-1-d) was obtained in the same manner as in Example 1, except that the crosslinked polymer (B-5-1) was used in place of the crosslinked polymer (B-1-1), and 3 g of a 5 wt% CHAPS (produced by Fujifilm Wako Pure Chemical Corporation) cyclohexane solution was used in place of 2 g of the 5 wt% lecithin (produced by Fujifilm Wako Pure Chemical Corporation; derived from soybean) cyclohexane solution.

The volume average particle size of the crosslinked polymer (C-5-1-d) was measured with a particle analyzer (CAMSIZER XT produced by Retsch) and found to be 133 um. The physiological saline absorption capacity was 30 g/g. The physiological saline solution pH was measured and found to be 7.4.

Example 11: Production of Crosslinked Polymer (C-6-1-d)

A crosslinked polymer (C-6-1-d) was obtained in the same manner as in Example 1, except that the crosslinked polymer (B-6-1) was used in place of the crosslinked polymer (B-1-1), and 2 g of a 5 wt% CHAPS (produced by Fujifilm Wako Pure Chemical Corporation) cyclohexane solution was used in place of 2 g of the 5 wt% lecithin (produced by Fujifilm Wako Pure Chemical Corporation; derived from soybean) cyclohexane solution.

The volume average particle size of the crosslinked polymer (C-6-1-d) was measured with a particle analyzer (CAMSIZER XT produced by Retsch) and found to be 630 um. The physiological saline absorption capacity was 33 g/g. The physiological saline solution pH was measured and found to be 7.8.

Example 12: Production of Crosslinked Polymer (C-4-0-a)

A crosslinked polymer (C-4-0-a) was obtained in the same manner as in Example 1, except that the crosslinked polymer (A-1) was used in place of the crosslinked polymer (B-1-1).

The volume average particle size of the crosslinked polymer (C-4-0-a) was measured with a particle analyzer (CAMSIZER XT produced by Retsch) and found to be 230 um. The physiological saline absorption capacity was 25 g/g. The physiological saline solution pH was measured and found to be 6.1.

Example 13: Production of Crosslinked Polymer (C-7-1-a)

A crosslinked polymer (C-7-1-a) was obtained in the same manner as in Example 1, except that the crosslinked polymer (B-7-1) was used in place of the crosslinked polymer (B-1-1).

The volume average particle size of the crosslinked polymer (C-7-1-a) was measured with a particle analyzer (CAMSIZER XT produced by Retsch) and found to be 220 um. The physiological saline absorption capacity was 3 g/g. The physiological saline solution pH was measured and found to be 7.4.

Example 14: Production of Crosslinked Polymer (C-8-1-a)

A crosslinked polymer (C-8-1-a) was obtained in the same manner as in Example 1, except that the crosslinked polymer (B-8-1) was used in place of the crosslinked polymer (B-1-1).

The volume average particle size of the crosslinked polymer (C-8-1-a) was measured with a particle analyzer

(CAMSIZER XT produced by Retsch) and found to be 220 um. The physiological saline absorption capacity was 100 g/g. The physiological saline solution pH was measured and found to be 7.4.

Example 15: Production of Crosslinked Polymer (C-9-1-a)

A crosslinked polymer (C-9-1-a) was obtained in the same manner as in Example 1, except that the crosslinked polymer (B-9-1) was used in place of the crosslinked polymer (B-1-1).
The volume average particle size of the crosslinked polymer (C-9-1-a) was measured with a particle analyzer (CAMSIZER XT produced by Retsch) and found to be 155 um. The physiological saline absorption capacity was 21 g/g. The physiological saline solution pH was measured and found to be 7.4.

Example 16: Production of Crosslinked Polymer (C-10-1-a)

A crosslinked polymer (C-10-1-a) was obtained in the same manner as in Example 1, except that the crosslinked polymer (B-10-1) was used in place of the crosslinked polymer (B-1-1).
The volume average particle size of the crosslinked polymer (C-10-1-a) was measured with a particle analyzer (CAMSIZER XT produced by Retsch) and found to be 297 μm. The physiological saline absorption capacity was 22 g/g. The physiological saline solution pH was measured and found to be 7.4.

Example 17: Production of Crosslinked Polymer (C-1-1-a2)

A crosslinked polymer (C-10-1-a2) was obtained in the same manner as in Example 1, except that 1 g of the 5 wt% lecithin (produced by Fujifilm Wako Pure Chemical Corporation; derived from soybean) cyclohexane solution was used in place of 2 g of the 5 wt% lecithin (produced by Fujifilm Wako Pure Chemical Corporation; derived from soybean) cyclohexane solution.
The volume average particle size of the crosslinked polymer (C-10-1-a2) was measured with a particle analyzer (CAMSIZER XT produced by Retsch) and found to be 222 μm. The physiological saline absorption capacity was 22 g/g. The physiological saline solution pH was measured and found to be 7.4.

Example 18: Production of Crosslinked Polymer (C-1-1-a3)

A crosslinked polymer (C-1-1-a3) was obtained in the same manner as in Example 1, except that 4 g of a 20 wt% lecithin (produced by Fujifilm Wako Pure Chemical Corporation; derived from soybean) cyclohexane solution was used in place of 2 g of the 5 wt% lecithin (produced by Fujifilm Wako Pure Chemical Corporation; derived from soybean) cyclohexane solution.
The volume average particle size of the crosslinked polymer (C-1-1-a3) was measured with a particle analyzer (CAMSIZER XT produced by Retsch) and found to be 231 μm. The physiological saline absorption capacity was 23 g/g. The physiological saline solution pH was measured and found to be 7.4.

Example 19: Production of Crosslinked Polymer (C-11-1-g)

A crosslinked polymer (C-11-1-g) was obtained in the same manner as in Example 1, except that the crosslinked polymer (B-11-1) was used in place of the crosslinked polymer (B-1-1), and 2 g of a 5 wt% polylysine (number average molecular weight: 4700) cyclohexane solution was used in place of 2 g of the 5 wt% lecithin (produced by Fujifilm Wako Pure Chemical Corporation; derived from soybean) cyclohexane solution.
The volume average particle size of the crosslinked polymer (C-11-1-g) was measured with a particle analyzer (CAMSIZER XT produced by Retsch) and found to be 302 μm. The physiological saline absorption capacity was 30 g/g. The physiological saline solution pH was measured and found to be 7.9.

Example 20: Production of Crosslinked Polymer (C-11-1-h)

A crosslinked polymer (C-11-1-h) was obtained in the same manner as in Example 1, except that the crosslinked polymer (B-11-1) was used in place of the crosslinked polymer (B-1-1), and 2 g of a 0.5 wt% polyethylenimine (number average molecular weight: 600) cyclohexane solution was used in place of 2 g of the 5 wt% lecithin (produced by Fujifilm Wako Pure Chemical Corporation; derived from soybean) cyclohexane solution.
The volume average particle size of the crosslinked polymer (C-11-1-h) was measured with a particle analyzer (CAMSIZER XT produced by Retsch) and found to be 288 μm. The physiological saline absorption capacity was 28 g/g. The physiological saline solution pH was measured and found to be 7.8.

Example 21: Production of Crosslinked Polymer (C-4-1-f)

A crosslinked polymer (C-4-1-f) was obtained in the same manner as in Example 1, except that the crosslinked polymer (B-4-1) was used in place of the crosslinked polymer (B-1-1), and 2 g of a 5 wt% triethanolamine (produced by Tokyo Chemical Industry Co., Ltd.) ethanol solution was used in place of 2 g of the 5 wt% lecithin (produced by Fujifilm Wako Pure Chemical Corporation; derived from soybean) cyclohexane solution.

The volume average particle size of the crosslinked polymer (C-4-1-f) was measured with a particle analyzer (CAMSIZER XT produced by Retsch) and found to be 241 um. The physiological saline absorption capacity was 27 g/g. The physiological saline solution pH was measured and found to be 7.5.

1 g of the crosslinked polymer (C) produced in each Example was placed on a Petri dish, covered with filter paper, and heat-dried in a circulating dryer at 130°C for 60 minutes, and the weight of the residue after heat drying was measured. The ratio of the weight of the crosslinked polymer (C) after heat drying to the weight of the crosslinked polymer (C) before heat drying (100 - loss on drying (%), where the loss on drying (%) means the percentage of weight loss of the polymer (C) when heat-dried relative to the weight of the polymer (C) before heat drying) is shown in Tables 1 to 4.

Test Example: Recovery of Extracellular Vesicle Using Crosslinked Polymer

Extracellular vesicles (exosomes) were separated and recovered by the following method using the crosslinked polymers produced in Examples 1 to 21 and the following crosslinked polymers for comparison, and the amount of extracellular vesicles recovered, the degree of purification, etc. were evaluated.

In Comparative Example 1, the crosslinked polymer (A-1) obtained in Production Example 1 was used as a crosslinked polymer for comparison.

In Comparative Example 2, the crosslinked polymer (B-1-1) obtained in Production Example 12 was used as a crosslinked polymer for comparison.

In Comparative Example 3, no crosslinked polymer was added, and the test was performed.

1 mL of each sample was centrifuged at 1200×g for 10 minutes, and the supernatant from which debris had been removed was collected. 40 mg of a crosslinked polymer was added to a 1.5-mL microtube, then 1 mL of the supernatant from which debris had been removed was added, and the mixture was allowed to stand at 25°C for 30 minutes. Thereafter, the supernatant was discarded, and a washing operation including adding 0.5 mL of physiological saline (sodium chloride concentration: 0.9 wt%) to the resulting gel, stirring the mixture by pipetting, and then discarding the supernatant was repeated three times.

Subsequently, 70 mg of sodium chloride was added, and the mixture was allowed to stand at 25°C for 30 minutes. The free liquid was then collected with a pipette, the collected liquid was sealed in a dialysis tube (produced by Fujifilm Wako Pure Chemical Corporation; Dialysis Membrane, size 20, MWCO: 14000), desalting was performed at 25°C for 2 hours, and extracellular vesicles were recovered.

The weight of the collected liquid, the amount of exosomes recovered per mL of the sample, and the amount of albumin recovered per mL of the sample are as shown in Tables 1 to 4.

The degree of purification was also calculated using the following method.

[Degree of purification] = [amount of exosomes recovered per mL of sample]/[amount of albumin recovered per mL of sample]

The amount of exosomes recovered was measured using an ELISA kit (produced by Cosmo Bio Co., Ltd.; CD9/CD63 Exosome ELISA KIT, Human). The amount of albumin recovered was measured using an ELISA kit (produced by Proteintech; Human Albumin ELISA Kit) .

In addition, for further comparison, ultracentrifugation, which is a conventional method, was performed, and the weight of the collected liquid, the amount of exosomes recovered per mL of the sample, the amount of albumin recovered per mL of the sample, and the degree of purification were evaluated in the same manner as above.

30 mL of a human urine sample was subjected to ultracentrifugation to isolate and recover exosomes. Specifically, centrifugation was continuously performed at 4°C as follows.

30 mL of a human urine sample was centrifuged at 1200×g for 10 minutes, and the supernatant was collected, followed by centrifugation at 10000×g for 30 minutes. Subsequently, the supernatant was collected, followed by centrifugation at 70000×g for 1 hour. The supernatant was then discarded. The sedimented pellet was resuspended in 5 mL of a 0.25 M sucrose solution (20 mM HEPES; pH 7.2), and the resulting suspension was added to a 40PA tube containing 30 mL of a 0.25 M to 2 M sucrose gradient solution (20 mM HEPES; pH 7.2), followed by centrifugation at 100000×g for 20 hours. The liquid in the 40PA tube was suctioned up from the bottom, and 2-mL aliquots of 8-mL to 12-mL fractions were placed into 5PA tubes each containing 3 mL of PBS(-). The liquid placed into the 5PA tubes was

centrifuged at 200000×g for 1 hour, the sedimented pellet was resuspended in 150 μL of PBS(-), and extracellular vesicles were recovered.

Tables 1 to 4 show the results.

Table 1

| | Ex. 1 | Ex. 2 | Ex. 3 | Ex. 4 | Ex. 5 | Ex. 6 | Ex. 7 |
|---|---|---|---|---|---|---|---|
| Crosslinked polymer | (C-1-1-a) | (C-1-2-a) | (C-1-3-a) | (C-1-4-a) | (C-2-1-a) | (C-2-1-b) | (C-3-1-c) |
| Coating agent | Lecithin 1 wt%/SAP | Lecithin 1 wt%/SAP | Lecithin 1 wt%/SAP | Lecithin 1 wt%/SAP | Lecithin 1.5 wt%/SAP | BIGCHAP 0.5 wt%/SAP | HDTMA-Cl 0.5 wt%/SAP |
| Particle size (μm) | 220 | 218 | 230 | 218 | 201 | 207 | 420 |
| Physiological saline absorption capacity | 22 | 23 | 19 | 22 | 11 | 11 | 32 |
| Physiological saline solution pH | 7.4 | 7.1 | 7.9 | 7.2 | 7.4 | 7.4 | 7.0 |
| Ratio of weight of crosslinked polymer (C) after heat drying to weight of crosslinked polymer (C) before heat drying | 98.7 | 97.9 | 97.6 | 98.6 | 98.9 | 96.7 | 97.4 |
| Molar concentration of cationic group relative to weight of crosslinked polymer after drying (mol/g) | 1.29E-05 | 1.30E-05 | 1.30E-05 | 1.29E-05 | 1.92E-05 | 0.00E+00 | 1.60E-05 |
| Sample | Healthy subject urine | Healthy subject urine | Healthy subject urine | Healthy subject urine | Healthy subject urine | Healthy subject urine | Healthy subject urine |
| Amount of exosomes recovered per mL of sample | 23.7 | 24 | 11.1 | 23.9 | 20.2 | 18.5 | 19.9 |
| Amount of albumin recovered per mL of sample | 0.8 | 1.8 | 0.5 | 1.2 | 1.1 | 2.3 | 2.6 |
| Degree of purification | 29.6 | 13.3 | 22.2 | 19.9 | 18.4 | 8.0 | 7.7 |

Table 2

| | Ex. 8-1 | Ex. 8-2 | Ex. 9 | Ex. 10-1 | Ex. 10-2 | Ex. 11-1 | Ex. 11-2 |
|---|---|---|---|---|---|---|---|
| Crosslinked polymer | (C-4-1-d) | (C-4-1-d) | (C-4-1-e) | (C-5-1-d) | (C-5-1-d) | (C-6-1-d) | (C-6-1-d) |
| Coating agent | CHAPS 1 wt%/SAP | CHAPS 1 wt%/SAP | Lauryl alcohol 1 wt%/SAP | CHAPS 1.5 wt%/SAP | CHAPS 1.5 wt%/SAP | CHAPS 1 wt%/SAP | CHAPS 1 wt%/SAP |
| Particle size (μm) | 222 | 222 | 228 | 133 | 133 | 630 | 630 |
| Physiological saline absorption capacity | 31 | 31 | 28 | 30 | 30 | 33 | 33 |
| Physiological saline solution pH | 7.5 | 7.5 | 7.4 | 7.4 | 7.4 | 7.8 | 7.8 |

(continued)

| | Ex. 8-1 | Ex. 8-2 | Ex. 9 | Ex. 10-1 | Ex. 10-2 | Ex. 11-1 | Ex. 11-2 |
|---|---|---|---|---|---|---|---|
| Ratio of weight of crosslinked polymer (C) after heat drying to weight of crosslinked polymer (C) before heat drying | 98.2 | 98.2 | 98.8 | 98.1 | 98.1 | 99.1 | 99.1 |
| Molar concentration of cationic group relative to weight of crosslinked polymer after drying (mol/g) | 1.64E-05 | 1.64E-05 | 0.00E+00 | 2.45E-05 | 2.45E-05 | 1.62E-05 | 1.62E-05 |
| Sample | Human serum | Healthy subject urine | Healthy subject urine | Human serum | Healthy subject urine | Human serum | Healthy subject urine |
| Amount of exosomes recovered per mL of sample | 36.3 | 22.2 | 4.8 | 31.8 | 18 | 28.5 | 12 |
| Amount of albumin recovered per mL of sample | 5.5 | 1.1 | 1.7 | 4.1 | 1.7 | 3.7 | 0.8 |
| Degree of purification | 6.6 | 20.2 | 2.8 | 7.8 | 10.6 | 7.7 | 15 |

Table 3

| | Ex. 12 | Ex. 13 | Ex. 14 | Ex. 15 | Ex. 16 | Ex. 17 | Ex. 18 |
|---|---|---|---|---|---|---|---|
| Crosslinked polymer | (C-4-0-a) | (C-7-1-a) | (C-8-1-a) | (C-9-1-a) | (C-10-1-a) | (C-1-1-a2) | (C-1-1-a3) |
| Coating agent | Lecithin 1 wt%/SAP | Lecithin 1 wt%/SAP | Lecithin 1 wt%/SAP | Lecithin 1 wt%/SAP | Lecithin 1 wt%/SAP | Lecithin 0.5 wt%/SAP | Lecithin 8 wt%/SAP |
| Particle size ($\mu$m) | 230 | 220 | 220 | 155 | 297 | 222 | 231 |
| Physiological saline absorption capacity | 25 | 3 | 100 | 21 | 22 | 22 | 23 |
| Physiological saline solution pH | 6.1 | 7.4 | 7.4 | 7.4 | 7.4 | 7.4 | 7.4 |
| Ratio of weight of crosslinked polymer (C) after heat drying to weight of crosslinked polymer (C) before heat drying | 99.1 | 99.5 | 94.2 | 98.8 | 98.5 | 98.6 | 98.8 |
| Molar concentration of cationic group relative to weight of crosslinked polymer after drying (mol/g) | 1.28E-05 | 1.28E-05 | 1.35E-05 | 1.28E-05 | 1.29E-05 | 6.46893E-06 | 9.61E-05 |

(continued)

| | Ex. 12 | Ex. 13 | Ex. 14 | Ex. 15 | Ex. 16 | Ex. 17 | Ex. 18 |
|---|---|---|---|---|---|---|---|
| Sample | Healthy subject urine | Healthy subject urine | Healthy subject urine | Healthy subject urine | Healthy subject urine | Healthy subject urine | Healthy subject urine |
| Amount of exosomes recovered per mL of sample | 22.2 | 18.1 | 29.1 | 25.1 | 21.1 | 21 | 19.8 |
| Amount of albumin recovered per mL of sample | 6.1 | 1.1 | 1.7 | 0.9 | 0.78 | 0.8 | 0.9 |
| Degree of purification | 3.6 | 16.5 | 17.1 | 27.9 | 27.1 | 26.3 | 22.0 |

Table 4

| | Ex. 19 | Ex. 20 | Ex. 21 | Comp. Ex. 1 | Comp. Ex. 2 | Comp. Ex. 3 |
|---|---|---|---|---|---|---|
| Crosslinked polymer | (C-11-1-g) | (C-11-1-h) | (C-4-1-f) | (A-1) | (B-1-1) | - |
| Coating agent | Polylysine (number average molecular weight: 4700) 1 wt%/SAP | Polyethylenimine (number average molecular weight: 600) 0.1 wt%/SAP | Triethanolamine 1 wt%/SAP | | | - |
| Particle size (μm) | 302 | 288 | 241 | 220 | 232 | - |
| Physiological saline absorption capacity | 30 | 28 | 27 | 26 | 22 | - |
| Physiological saline solution pH | 7.9 | 7.8 | 7.5 | 6.1 | 7.5 | - |
| Ratio of weight of crosslinked polymer (C) after heat drying to weight of crosslinked polymer (C) before heat drying | 98.3 | 98.4 | 98.5 | 98.5 | 98.2 | - |
| Molar concentration of cationic group relative to weight of crosslinked polymer after drying (mol/g) | 6.85E-05 | 2.36E-05 | 9.95E-05 | | | |
| Sample | Healthy subject urine | Healthy subject urine | Healthy subject urine | Healthy subject urine | Healthy subject urine | Healthy subject urine |
| Amount of exosomes recovered per mL of sample | 19 | 20.1 | 10.4 | 2.4 | 1.5 | 0.9 |
| Amount of albumin recovered per mL of sample | 3.1 | 3.5 | 3.5 | 6.3 | 0.8 | 3.4 |

(continued)

| | Ex. 19 | Ex. 20 | Ex. 21 | Comp. Ex. 1 | Comp. Ex. 2 | Comp. Ex. 3 |
|---|---|---|---|---|---|---|
| Degree of purification | 6.1 | 5.7 | 3 | 0.4 | 1.9 | 0.3 |

Industrial Applicability

The crosslinked polymer of the present invention can be suitably used for separating a substance having a lipid bilayer from a sample of biological origin. The separation method of the present invention increases the purity of the separated substance having a lipid bilayer and decreases the operation time as compared with, for example, conventional ultracentrifugation. The separation method of the present invention also decreases the number of steps as compared with, for example, conventional ultracentrifugation, and is also excellent in reproducibility. The separated substance having a lipid bilayer is usable in evaluation of the condition of the original organism (e.g., testing or diagnosis). The separated substance having a lipid bilayer is also usable as a carrier for cellularly targeted therapeutic agents.

## Claims

1. A crosslinked polymer for separating a substance having a lipid bilayer from a sample of biological origin, the crosslinked polymer comprising a monomer unit containing an acidic group and/or a neutralized salt group thereof, the crosslinked polymer comprising a compound containing at least one group selected from the group consisting of a cationic group and a hydroxyl group.

2. The crosslinked polymer according to claim 1, wherein the compound contains an optionally substituted $C_{12-30}$ hydrocarbon group.

3. The crosslinked polymer according to claim 1 or 2, wherein the compound contains a cationic group.

4. The crosslinked polymer according to any one of claims 1 to 3, wherein the cationic group is $-N(R^1)_2$ (wherein each $R^1$ independently represents a hydrogen atom or an optionally substituted $C_{1-6}$ hydrocarbon group) or $-N^+(R^2)_3$ (wherein each $R^2$ independently represents a hydrogen atom or an optionally substituted $C_{1-6}$ hydrocarbon group) .

5. The crosslinked polymer according to any one of claims 1 to 4, wherein the $C_{12-30}$ hydrocarbon group is a $C_{12-20}$ aliphatic hydrocarbon group or a $C_{20-30}$ alicyclic hydrocarbon group.

6. The crosslinked polymer according to claim 5, wherein the aliphatic hydrocarbon group is an alkyl group or an alkenyl group, and the alicyclic hydrocarbon group is a group having a steroid skeleton.

7. The crosslinked polymer according to any one of claims 1 to 6, wherein the pH of a physiological saline solution containing the crosslinked polymer in an amount of 0.5 wt% based on the weight of physiological saline is 7 to 8.

8. The crosslinked polymer according to any one of claims 1 to 7, which has a volume average particle size of 100 to 1000 um.

9. The crosslinked polymer according to any one of claims 1 to 8, wherein the substance having a lipid bilayer is an extracellular vesicle.

10. A method for separating a substance having a lipid bilayer from a sample of biological origin, comprising the following steps (1) and (2):

    (1) bringing a sample of biological origin into contact with the crosslinked polymer according to any one of claims 1 to 9 to obtain a polymer gel containing a substance having a lipid bilayer; and
    (2) mixing the polymer gel with a salt to recover the substance having a lipid bilayer.

11. A kit for separating a substance having a lipid bilayer from a sample of biological origin, comprising the crosslinked polymer according to any one of claims 1 to 9 and a salt.

**12.** A disease test kit using an extracellular vesicle, comprising the crosslinked polymer according to any one of claims 1 to 9 and a salt.

**13.** A device for separating a substance having a lipid bilayer from a sample of biological origin, comprising a means for performing the method according to claim 10.

**INTERNATIONAL SEARCH REPORT**

International application No. **PCT/JP2022/016441**

**A. CLASSIFICATION OF SUBJECT MATTER**

***C12M 1/00***(2006.01)i; ***G01N 33/48***(2006.01)i; ***G01N 33/50***(2006.01)i
FI: G01N33/48 A; G01N33/50 Z; C12M1/00 A

According to International Patent Classification (IPC) or to both national classification and IPC

**B. FIELDS SEARCHED**

Minimum documentation searched (classification system followed by classification symbols)

C12M1/00; G01N33/48; G01N33/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2019-518428 A (UNICITY E. V. AG) 04 July 2019 (2019-07-04) entire text, all drawings, in particular, see claims, paragraphs [0008]-[0014], etc. | 1-4, 9-13 |
| X | WO 2020/080387 A1 (MIRAKA CENTRAL RABORATORIES G.K.) 23 April 2020 (2020-04-23) entire text, all drawings, in particular, see claims, paragraphs [0010]-[0050], etc. | 1, 3-4, 9-13 |
| A | JP 2020-502067 A (EXOFARMA LTD.) 23 January 2020 (2020-01-23) see entire text, all drawings, etc. | 1-13 |
| A | JP 2021-007915 A (SHOWA DENKO MATERIALS CO., LTD.) 28 January 2021 (2021-01-28) see entire text, etc. | 1-13 |
| A | JP 2884063 B2 (DIRECTOR OF AGENCY OF INDUSTRIAL SCIENCE AND TECHNOLOGY) 19 April 1999 (1999-04-19) see entire text, etc. | 1-13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 June 2022** | **21 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/016441**

**C. DOCUMENTS CONSIDERED TO BE RELEVANT**

| Category* | Citation of document, with indication, where appropriate, of the relevant passages | Relevant to claim No. |
|---|---|---|
| A | JP 2001-519836 A (CENTRE NATIONAL DE LA RECHERCHE SCIENTIFIQUE (C.N.R.S.)) 23 October 2001 (2001-10-23) see entire text, etc. | 1-13 |
| P, X | WO 2021/251462 A1 (TOKUSHIMA UNIVERSITY) 16 December 2021 (2021-12-16) entire text, in particular, see claims, etc. | 1-4, 8-13 |

Form PCT/ISA/210 (second sheet) (January 2015)

**INTERNATIONAL SEARCH REPORT**
**Information on patent family members**

International application No.
**PCT/JP2022/016441**

| Patent document cited in search report | Publication date (day/month/year) | Patent family member(s) | Publication date (day/month/year) |
|---|---|---|---|
| JP 2019-518428 A | 04 July 2019 | US 2020/0179827 A1<br>entire text, all drawings, in particular, see claims, paragraphs [0016]-[0035], etc.<br>WO 2017/178472 A1<br>EP 3443112 A1 | |
| WO 2020/080387 A1 | 23 April 2020 | US 2021/0396633 A1<br>entire text, all drawings, in particular, see claims, paragraphs [0025]-[0073], etc.<br>EP 3869177 A1 | |
| JP 2020-502067 A | 23 January 2020 | US 2020/0023012 A1<br>see entire text, all drawings, etc.<br>US 2021/0128632 A1<br>WO 2018/112557 A1<br>EP 3559205 A1 | |
| JP 2021-007915 A | 28 January 2021 | (Family: none) | |
| JP 2884063 B2 | 19 April 1999 | (Family: none) | |
| JP 2001-519836 A | 23 October 2001 | WO 1998/027434 A1<br>see entire text, etc.<br>EP 946875 A1 | |
| WO 2021/251462 A1 | 16 December 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003225565 A
- JP 2005075982 A
- JP 3648553 B
- JP 2003165883 A
- JP 2005095759 A